# EUROPÄISCHE PATENTSCHRIFT

(11) **EP 1 165 061 B1**
(45) Veröffentlichungstag und Bekanntmachung des Hinweises auf die Patenterteilung: **19.10.2005**
(21) Anmeldenummer: 00929342.4
(22) Anmeldetag: 06.04.2000
(51) Int. Cl.: A61K 31/135, A61K 31/445, A61K 31/565, A61P 19/10

(54) **VERWENDUNG EINES KOMBINATIONSPRÄPARATES AUS VITAMIN-D-METABOLITEN ODER VITAMIN-D-ANALOGA UND EINES ÖSTROGENPARTIALAGONISTEN ZUR BEHANDLUNG VON OSTEOPOROSE**
USE OF COMPOUND PREPARATION MADE OF VITAMIN D METABOLITES OR VITAMIN D ANALOGUES AND PARTIAL ESTROGEN AGONISTS FOR TREATING OSTEOPOROSIS
UTILISATION D' UNE PREPARATION COMBINEE ASSOCIANT DES METABOLITES DE VITAMINE D OU DES ANALOGUES DE VITAMINE D ET DES AGONISTES PARTIELS D'OESTROGENE POUR LE TRAITEMENT DE L'OSTEOPOROSE

(30) Priorität: 08.04.1999 DE 19916419
(43) Veröffentlichungstag der Anmeldung: 02.01.2002
(73) Patentinhaber: Schering Aktiengesellschaft, 13353 Berlin (DE)
(72) Erfinder: KNAUTHE, Rudolf, D-13467 Berlin (DE); ERBEN, Reinhold, D-85635 Höhenkirchen (DE); BEHRENS-STEVENS, Marie-Luise, D-10623 Berlin (DE)
(86) Internationale Anmeldenummer: PCT/EP2000/003079
(87) Internationale Veröffentlichungsnummer: WO 2000/061123

(56) Entgegenhaltungen:
- WO-A-94/00128
- WO-A-99/12894
- GB-A- 2 269 176
- US-A- 5 403 824
- DATABASE WPI Section Ch, Week 198927 Derwent Publications Ltd., London, GB; Class B05, AN 1989-195622 XP002148536 & JP 01 132524 A (TEIJIN LTD), 25. Mai 1989 (1989-05-25)
- VINK-VAN WIJNGAARDEN TRUDY ET AL: "Inhibition of breast cancer cell growth by combined treatment with vitamin D-3 analogues and tamoxifen." CANCER RESEARCH, Bd. 54, Nr. 21, 1994, Seiten 5711-5717, XP000937998 ISSN: 0008-5472
- RIGGS B L ET AL: "EFFECT OF THE FLUORIDE CALCIUM REGIMEN ON VERTEBRAL FRACTURE OCCURRENCE IN POST MENOPAUSAL OSTEO POROSIS COMPARISON WITH CONVENTIONAL THERAPY" NEW ENGLAND JOURNAL OF MEDICINE, Bd. 306, Nr. 8, 1982, Seiten 446-450, XP000937673 ISSN: 0028-4793
- SOMJEN DALIA ET AL: "Nonhypercalcemic analogs of vitamin D stimulate creatine kinase B activity in osteoblast-like ROS 17/2.8 cells and up-regulate their responsiveness to estrogens." STEROIDS, Bd. 63, Nr. 5-6, Mai 1998 (1998-05), Seiten 340-343, XP000937674 ISSN: 0039-128X
- SOMJEN D ET AL: "Pretreatment with 1,25(OH)-2 vitamin D or 24,25(OH)-2 vitamin D increases synergistically responsiveness to sex steroids in skeletal-derived cells." JOURNAL OF STEROID BIOCHEMISTRY AND MOLECULAR BIOLOGY, Bd. 55, Nr. 2, 1995, Seiten 211-217, XP000938135 ISSN: 0960-0760
- BURCKHARDT P: "Selective estrogen receptor modulators (SERM): New substances for hormone replacement therapy." SCHWEIZERISCHE MEDIZINISCHE WOCHENSCHRIFT, Bd. 129, Nr. 49, 11. Dezember 1999 (1999-12-11), Seiten 1926-1930, XP000937653 ISSN: 0036-7672

## Beschreibung

Die Erfindung betrifft die Verwendung eines Kombinationspräparats aus einem Knochenzellaktivator und einem Resorptionsinhibitor zur Osteoporosebehandlung.
Osteoporose stellt die häufigste Form der metabolischen Knochenerkrankungen dar und ist charakterisiert durch eine gegenüber der Norm verminderte Knochenmasse, durch eine Veränderung der Mikroarchitektur des Knochens und ein vermehrtes Auftreten von Knochenfrakturen. Die Osteoporose läßt sich grob unterteilen in eine postmenopausale Form, die Frauen nach Erlöschen der Ovarfunktion erleiden und eine senile Form, die Frauen und Männer betrifft, die älter als ca. 65-70 Jahre sind. Die Prophylaxe und auch Therapie der Osteoporose werden heutzutage vor allem mit resorptionshemmenden Pharmaka (z. B. Östrogene, Calcitonin, Biphosphonate) durchgeführt, die einen weiteren Knochenverlust verhindern können. Östrogene werden in Form einer Monotherapie eingesetzt, was aber zu unerwünschten proliferativen Effekten auf den Uterus führt, oder in Form einer Kombinationstherapie zusammen mit einem Gestagen. Nachteil dieser kombinierten Therapie ist die geringe Kompliance, da die wiedereinsetzende Monatsblutung für viele Frauen nicht akzeptabel ist. Allen resorptionshemmenden Prinzipien, einschließlich der Östrogentherapie ist gemein, daß man durch sie vorhandene Knochenmasse erhalten kann, aber bereits verlorenen Knochensubstanz nicht wieder emeuert werden kann. Neuere Ansätze versuchen in den Prozeß des "Remodeling" einzugreifen. Remodeling ist ein zyklischer Vorgang, bei dem zunächst ein Quantum Knochensubstanz durch knochenresorbierende Zellen (Osteoklasten) abgebaut wird und danach - zeitlich gekoppelt an diesen Resorptionsvorgang- an der gleichen Stelle durch knochenaufbauende Zellen (Osteoblasten) wieder neu aufgebaut wird. Bei jungen, gesunden Menschen wird genauso viel Knochen neu aufgebaut wie vorher resorbiert wurde. Bei der Osteoporose ist das Knochenremodeling gestört. Ein theoretisches Modell (Frost HM 1979, Clin. Orthop. Rcl. Res. 143, 227-244) beschreibt die Erhöhung der Knochenmasse durch (i) Synchronisierung von Remodeling-Einheiten durch knochenzellaktivierende Substanzen, (ii) anschließende temporäre Hemmung der Knochenresorption und (iii) ein darauffolgendes therapiefreies Intervall. Ein Ansatz (EP 0381296) beschreibt die Verwendung eines resorptionsinhibierenden Polyphosphonats zusammen mit einem Knochenzellstimulator entsprechend diesem theoretischen Modell. Eine klinische Studie an Osteoporose-Patientinnen (Ott et al. 1994, J. Clin. Endocr. Metabol. 78, 968-972) hat jedoch gezeigt, daß die in dem Patent EP 0381296 beschriebene Therapie-Form nicht zu einem anabolen Effekt auf die Knochenmasse und auch zu keiner Zunahme der Aktivierungsfrequenz von Remodeling-Einheiten führte. Tierexperimentelle Untersuchungen (Delmas et al. 1995, Bone 16, 603-610) weisen darauf hin, daß ein derartiges Therapieregime unter Vewendung von Polyphosphonaten als knochenresorptionshemmende Substanzen nicht zu einer Zunahme der Knochenmasse führt, weil die lange Verweildauer und damit langanhaltende antiresorptive Wirkung der Polyphosphonate anabole Effekte am Knochen unterdrückt, die auf einer Beeinflussung von neu initiierten Remodeling-Einheiten beruhen.

Das Problem ist deshalb, eine Kombination eines Knochenzellaktivators und eines Resorptionsinhibitors zu finden, die so in den Prozeß des Remodeling eingreift, daß eine Zunahme der Knochenmasse resultiert.

Das Problem wird durch ein Kombinationspräparat gelöst, bei dem der Knochenzellaktivator ein Vitamin-D-Metabolit oder ein Vitamin-D-Analogon und der Resorptionsinhibitor ein Östrogenpartialagonist ist und das in einem Therapieplan verwendet wird, welcher einen oder mehrere Zyklen umfaßt, wobei jeder Zyklus aus folgenden Schritten besteht:
a) tägliche Verabreichung eines Vitamin-D-Metaboliten oder Vitamin-D-Analogons für 1-7 Tage.
b) anschließend daran oder nach einer Ruhephase bis zu 30 Tagen eine tägliche Verabreichung ein Östrogenpartialagonist für 21-120 Tage.

Mit Kombinationspräparat ist gemeint, daß der Vitamin-D-Metabolit oder das Vitamin-D-Analogon und ein Östrogenpartialagonist in der gleichen Applikationsform oder in unterschiedlichen Applikationsformen in einem Arzneimittelpaket bereitgestellt werden. Für ein therapiefreies Intervall können auch Placebos dem Arzneimittelpaket zugefügt werden. Ein Östrogenpartialagonist ist eine Substanz, die an den Östrogenrezeptor bindet und mit dem natürlichen Liganden 17-β Östradiol um die Bindung kompetitiert. In Gegenwart des natürlichen Liganden wirkt der Partialagonist der Wirkung des Östrogens entgegen und unterbindet diese. Im Unterschied zu einem reinen Antagonisten wirkt der Partialagonist in Abwesenheit des natürlichen Liganden selbst teilweise oder vollständig als Östrogen. Die östrogene bzw. antiöstrogene Wirkung eines Partialagonisten läßt sich beispielsweise im Uteruswachstumstest oder Antiuteruswachstumstest in der Ratte oder der Maus *in vivo* messen (siehe Beispiel 2). Die bevorzugten Östrogenpartialagonisten zeigen eine selektive östrogene Wirkung vor allem auf den Knochenmetabolismus, dagegen nur eine geringe oder keine Wirkung auf den Uterus. Es stellte sich überraschenderweise heraus, daß die Effekte von 1α,25-Dihydroxyvitamin D₃ (1,25 VitD₃) und einem Östrogenpartialagonisten auf die Knochenmasse synergistisch sind. In einer Dosierung, bei der 1,25 VitD₃ allein und der Östrogenpartialagonist allein nur einen weiteren Knochenmassenverlust verhindern können, ist durch die Behandlung mit der Kombination von 1,25 VitD₃ und Östrogenpartialagonist eine deutliche Zunahme der Knochenmasse zu beobachten (siehe Beispiel 1 mit 1.25-Dihydroxyvitamin D3 und Raloxifen). Für diese Anwendung geeignete Östrogenpartialagonisten sind beispielsweise Raloxifen, Tamoxifen, Hydroxytamoxifen, Clomiphencitrat, Droloxifen, Idoxifen, Centchroman, Levormeloxifen, Cyclophenil, EM800 und CP336156. Bevorzugt sind 14α,17α-Ethano-11β-{4-[5-(2-pyridinmethylsulfonyl)pentyloxy]phenyl}-1,3,5(10)-estratrien-3,17β-diol und 14α,17α-Ethano-11β-{4-[5-(2-pyridinmethylsulfinyl)pentyloxy]phenyl}-1,3,5(10)-estratrien-3,17β-diol und weitere Verbindungen wie sie in DE19724187.5 beschrieben sind und Benzocycloheptene gemäß DE19833786.8 wie z.B. (4,4,5,5,5-Pentafluorpentyl)-{5-[4-(6-phenyl-8,9-dihydro-7H-benzocyclohepten-5-yl)-phenoxy]-pentyl}-sulfid, S-{5-[4-(6-Phenyl-8,9-dihydro-7H-benzocyclohepten-5-yl)-phenoxy]-pentyl}-thioacetat, 5-{4-[5-(4,4,5,5,5-Pentafluor-pentansulfinyl)-pentyloxy]-phenyl}-6-phenyl-8,9-dihydro-7H-benzocyclohepten-2-ol, N-Butyl-N-methyl-2-{5-[4-(2-hydroxy-6-phenyl-8,9-dihydro-7H-benzocyclohepten-5-yl)-phenoxy]-pentansulfinyl}-acetamid, 6-(4-Hydroxy-phenyl)-5-{4-[5-(4,4,5,5,5-pentafluor-pentansulfonyl)-pentyloxy]-phenyl}-8,9-dihydro-7H-benzocyclohepten-2-ol, 6-(4-Hydroxy-phenyl)-5-{4-[5-(4,4,5,5,5-pentafluorpentansulfinyl)-pentyloxy]-phenyl}-8,9-dihydro-7H-benzocyclohepten-2-ol, 5-{4-[4-(4,4,5,5,5-Pentafluor-pentansulfinyl)-butyloxy]-phenyl}-6-phenyl-8,9-dihydro-7H-benzocyclohepten-2-ol, 6-Phenyl-5-{4-[6-(pyridin-2-ylmethansulfinyl)-hexyloxy]-phenyl}-8,9-dihydro-7H-benzocyclohepten-2-ol. 5-{4-[5-(Furan-2-ylmethansulfinyl)-pentyloxy]-phenyl}-6-phenyl-8,9-dihydro-7H-benzocyclohepten-2-ol, 5-{4-[5-(3,3,4,4,5,5,5-Heptafluor-pentylthio)-pentyloxy]-phenyl}-6-phenyl-8,9-dihydro-7H-benzocyclohepten-2-ol, 6-Phenyl-5-{4-[5-(4,4,4-trifluor-butylthio)-pentyloxy]-phenyl}-8,9-dihydro-7H-benzocyclohepten-2-ol, 5-[4-(5-Benzylsulfinyl-pentyloxy)-phenyl]-6-phenyl-8,9-dihydro-7H-benzocyclohepten-2-ol, 6-Phenyl-5-{4-[5-(4-trifluormethyl-benzylsulfinyl)-pentyloxy]-phenyl}-8.9-dihydro-7H-benzocyclohepten-2-ol. 5-{4-[5-(4-tert.-Butyl-phenylthio)-pentyloxy]-phenyl}-6-phenyl-8,9-dihydro-7H-benzocyclohepten-2-ol und 6-Phenyl-5-{4-[5-(4-trifluormethyl-phenylsulfinyl)-pentyloxy]-phenyl}-8,9-dihydro-7H-benzocyclohepten-2-ol.

Weitere Beispiele sind publiziert (von Angerer E. (1995) Compounds acting as mixed agonists/antagonists. In: von Angerer E (ed) The estrogen receptor as a target for rational drug design. Springer, New York, pp 50 -95). Weiterhin bevorzugt sind Östrogenpartialagonisten, die im Uteruswachstumstest weniger als 50 % der Wirkung von der Substitutionsdosis von 17-β Östradiol haben und im Test auf Knochenwirkung mindestens 60% der Wirkung einer Substitutionsdosis von 17-β Östradiol am Knochen haben. Die Durchführung dieser Tests ist im Beispiel 2 beschrieben.

Für diese Anwendung geeignete Vitamin-D-Metaboliten oder Vitamin-D-Analoga sind 25-Hydroxyvitamin D₃ oder D₂, 1α,25-Dihydroxyvitamin D₃ oder D₂, 24R,25-Dihydroxyvitamin D₃, 1α,24R,25-Trihydroxyvitamin D₃, 1α-Hydroxyitamin D₃ oder D₂, 1α-24S-Dihydroxyvitamin D2, 1α,24R-Dihydroxyvitamin D3 oder D2, 24-Epi-1α-Hydroxyvitamin D₂, 24-Epi-1α,25-Dihydroxy-Vitamin D₂-19-nor-vitamin D₂, 22-Oxa-1α,25-Dihydroxyvitamin D₃, 2β-(3-Hydroxypropyl)-1α,25-Dihydroxyvitamin D₃, 1α,25-Dihydroxy-16-en-23-in-vitamin D₃, 1α,25-Dihydroxy-26,26,26,27,27,27-hexafluor-16-en-23-vitamin D₃, (5Z,7E,22E,24E)-(1S,3R)-24a,26,27-Trihomo-9,10-secopregna-5,7,10(19),22,24-pentaen-1,3,25-triol (EB 1089), (5Z,7E,22E)-(1S,3R,24R)-26,27-Cyclo-9,10-secocholesta-5,7,10(19),22-tetraen-1,3,24-triol (MC 903), oder 20-Epi-Derivate wie zum Beispiel (5Z,7E)-(1S,3R,20R)-20-(2-Ethyl-2-hyddroxyhexoxy)9,10-secopregna-5,7,10-(19)-trien-1,3-diol (KH 1060). Weitere Beispiele für Vitamin-D-Metaboliten oder Vitamin-D-Analoga sind publiziert (Bouillon R. et al. 1995, Endocrine Rev. 16, 200-257) Bevorzugt ist die Verwendung von 1α,25-Dihydroxyvitamin D₃ (siehe auch WO 94/00128).

Die Erfindung betrifft die Verwendung des erfindungsgemäßen Kombinationspräparats zur Herstellung eines Arzneimittels zur Behandlung oder Vorbeugung von postmenopausaler, seniler und sekundärer Osteoporose und zur Hormonersatztherapie. Dieses kann sowohl bei der Frau als auch beim Mann angewendet werden.

Der Vitamin-D-Metabolit oder das Vitamin-D-Analogon und der Partialagonist können oral, subkutan, transdermal, intravenös oder als Implantat appliziert werden. Dabei sind alle möglichen Kombinationen der Applikationsformen für Vitamin-D-Metaboliten oder Vitamin-D-Analoga und Partialagonisten möglich. Hierbei können verschiedene Formulierungen verwendet werden wie beispielsweise Tabletten, Tabletten mit kontrollierter Freisetzung des Wirkstoffs, Dragees, Pillen, Kapseln, Filmtabletten und Filmtabletten mit kontrollierter Freisetzung des Wirkstoffs, Pulver oder Depotformen sowie Suppositorien.

### Abbildungen

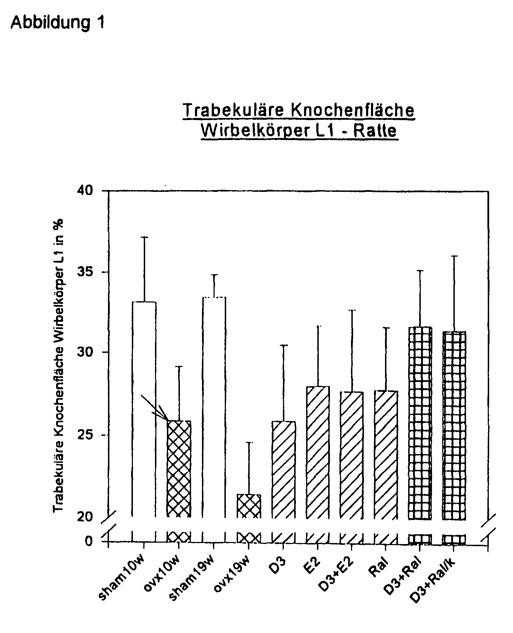

**Abbildung 1** zeigt die trabekuläre Knochenfläche des Wirbelkörpers L1 der Ratte.
Es bedeuten:
- sham10w:: Kontrollgruppe, 10 Wochen nach einer Scheinoperation
- ovx10w:: ovarektomierte Gruppe, 10 Wochen nach Operation
- sham19w:: Kontrollgruppe, 19 Wochen nach Scheinoperation
- ovx19w:: ovarektomierte Gruppe, 19 Wochen nach Operation
- D3:: ovarektomierte Gruppe, behandelt mit 0,2 µg/kg s.c. 1,25 VitD₃, 19 Wochen nach Operation
- E2:: ovarektomierte Gruppe, behandelt mit 0,3 µg/kg s.c. 17βÖstradiol, 19 Wochen nach Operation,
- D3+E2:: ovarektomierte Gruppe, behandelt mit 0,2 µg/kg s.c. 1,25 VitD₃ und 0,3 µg/kg s.c. Östradiol, 10 Wochen nach Operation,
- Ral:: ovarektomierte Gruppe, behandelt mit 2,5 mg/kg p.o. Raloxifen, 19 Wochen nach Operation
- D3+Ral:: ovarektomierte Gruppe, behandelt 0,2 µg/kg s.c. 1,25 VitD₃ und 2,5 mg/kg p.o. Raloxifen, 19 Wochen nach Operation, Behandlung erst mit 1,25 VitD₃, anschließend mit Raloxifen
- D3+Ral/k:: ovarektomierte Gruppe, behandelt 0,2 µg /kg s.c. 1,25 VitD₃ und 2,5 mg/kg p.o. Raloxifen, 19 Wochen nach Operation, kontinuierliche Behandlung mit Raloxifen

Die nachstehengen Beispiele dienen der näheren Erläuterung der Erfindung.

### Beispiel 1:

### Einfluß der Behandlung auf die trabekuläre Knochenfläche in Wirbelkörpern der ovarektomierten Ratte

### Experimentelle Vorgehensweise:

3 Monate alte weibliche Ratten wurden 10 Wochen vor Behandlungsbeginn sham operiert (Scheinoperation) oder ovariektomiert. Die Tiere der Gruppen 1 und 2 wurden unmittelbar vor Behandlungsbeginn als sham oder ovx Baselinekontrolle getötet. Die sham Gruppe 3 und die ovx Gruppe 4 wurden als unbehandelte Kontrollen im Experiment weiter geführt und mit entsprechenden Vehikeln behandelt. Die Tiere der Behandlungsgruppen (Gruppen 5 - 10) wurden über drei Zyklen behandelt, wobei ein Behandlungsintervall jeweils 3 Wochen (21 Tage) umfaßt. Gruppe 10 wurde wie Gruppe 9 behandelt, allerdings mit dem Unterschied, daß der Partialagonist Raloxifen durchgängig verabreicht wurde. Die Behandlungszyklen pro Gruppe waren wie folgt aufgebaut:

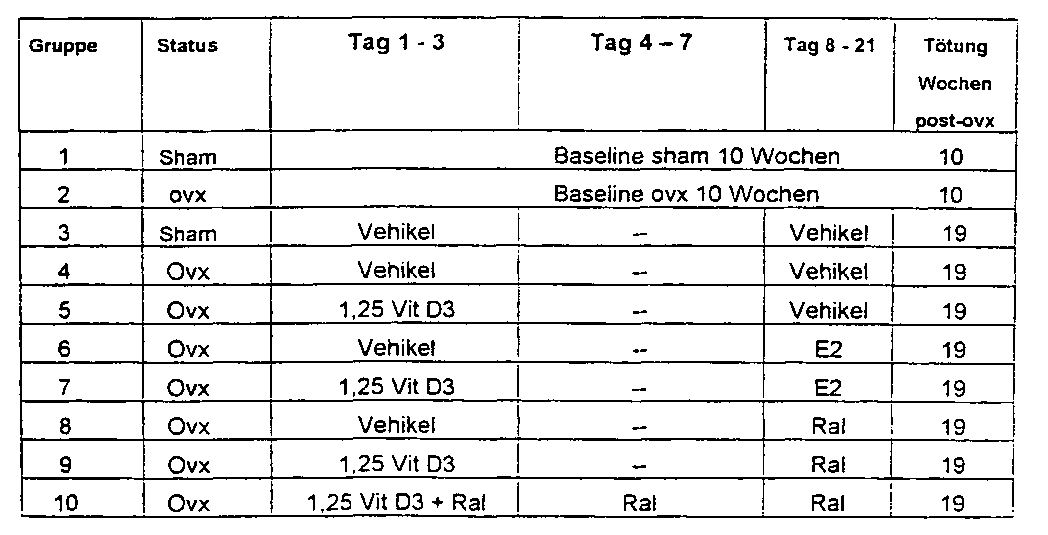

Nach dem Ende des dritten Zyklus wurden alle Tiere getötet und die Lendenwirbel L1 entnommen. Effekte auf den trabekulären Knochen wurden mittels quantitativer Histomorphometrie an nicht entkalkten Knochenschnitten erfaßt.

### Ergebnis:

Im Vergleich zu den sham Gruppen 1 und 3 (sham10w und sham19w) kann ein zeitabhängiger Verlust an trabekulärer Knochenmasse bei ovx-Tieren beobachtet werden (vergleiche in Abb.1 Knochenflächen der Gruppen 2 und 4 [ovx10w, ovx19w] mit Gruppen 1 und 3 [sham10w, sham19w]). Durch die isolierte Behandlung mit dem Knochenzellaktivator 1,25-Vit D3, oder mit den Östrogenkomponenten E2 oder Raloxifen kann der weitere Knochenmassenverlust aufgehalten werden (vergleiche ovx19w mit D3, E2 und Ral). Im Gegensatz zur Behandlung mit Raloxifen oder 1, 25 Vit D3 alleine führt die Behandlung mit einer Kombination aus beiden Wirkstoffen zu einer Zunahme der trabekulären Knochenmasse über das Ausgangsniveau hinaus (vergleiche ovx 10 w mit D3 +Ral und D3+ Ral/k).
Dies bedeutet, daß, im Unterschied zu der knochenerhaltenden Behandlung mit Östradiol, 1,25 Vitamin D3 oder Raloxifen allein, die Behandlung mit der Kombination in dem beschriebenen Therapieplan einen Zugewinn an neuer Knochenmasse bewirkt.

### Beispiel 2

### Pharmakologische Charakterisierung von Östrogenrezeptorpartialagonisten:

### 1. Uteruswachstumstest

Immature 19-21 Tage alte weibliche Ratten mit einem mittleren Körpergewicht von 35 - 50 Gramm werden an drei aufeinanderfolgenden Tagen mit dem zu testenden ÖstrogenPartialagonisten oder Vehikel behandelt. Als Vehikel können beispielsweise ölige Lösungen aus 10% Ethanol + 90 % Arachisöl oder ein Mischung von Benzylbenzoat/ Rizinusöl (1+4; v/v) verwendet werden. Am Tag 4 nach Versuchsbeginn werden die Versuchstiere betäubt und getötet. Die Uteri werden entnommen und das Feuchtgewicht durch Wägung bestimmt. Anschließend werden die Uteri bei 60°C für 4 h getrocknet und die Trockengewichte bestimmt. Das Versuchsergebnis wird ausgedrückt als relative Feucht- bzw. Trockengewichte der gewogenen Uteri in mg/100g Körpergewicht. Als negative Kontrolle dienen mit Vehikel behandelte immature Ratten. Als positive Kontrolle werden immature weibliche Ratten mit 0,3 µg/Tier/Tag 17-β Estradiol s.c. behandelt. Die Differenz der Uterigewichte von Tieren, die mit Vehikel oder 17-β Östradiol behandelt wurden, wird als 100 % definiert. Die östrogene Wirksamkeit eines Partialagonisten wird ausgedrückt als Prozent der Wirkung von 17-β Östradiol für die jeweils getestet Dosierung.

Im gleichen Versuchsdesign können auch adulte ovarektomiert Ratten oder Mäuse verwendet werden. Vor Versuchsbeginn sollten die Tier allerdings nach Ovarektomie 10-14 Tage ruhen, um die endogenen Hormonspiegel abzusenken.

### 2. Antiuteruswachstumstest

Der Uterus infantiler, östrogensubstituierter Ratten kann als Testmodell genutzt werden, um eine direkte Wirkung von Substanzen mit antiöstrogenen Eigenschaften nachzuweisen. Wie im Uteruswachstumstest beschrieben (s.o.) ist der Parameter für östrogene Wirkung bei infantilen Ratten die durch 17-β Östradiol induzierte Zunahme des Uterusgewichtes. Im Antiuteruswachstumstest wird diese Zunahme durch gleichzeitige Gabe einer antiöstrogen wirksamen Substanz gehemmt.
Immature 19 - 21 Tage alte weibliche Ratten mit einem mittleren Körpergewicht von 35 - 50 Gramm werden an drei aufeinanderfolgenden Tagen mit einer Kombination aus einer Substitutionsdosis von 0,3 µg/Tier/Tag 17-β Östradiol und der Testsubstanz s.c. behandelt. Als Vehikel können beispielsweise ölige Lösungen aus 10% Ethanol + 90 % Arachisöl oder ein Mischung von Benzylbenzoat/ Rizinusöl (1+4; v/v) verwendet werden. Am Tag 4 nach Versuchsbeginn werden die Versuchstiere betäubt und getötet. Die Uteri werden entnommen und das Feuchtgewicht durch Wägung bestimmt.
Als Positivkontrolle dient eine Gruppe von Tieren, die nur mit 0,3 µg 17-β Östradiol/Tier/Tag s.c behandelt wird, als Negativkontrolle eine mit Vehikel behandelte Gruppe von Tieren.
Das Versuchsergebnis wird ausgedrückt als relatives Feuchtgewichte der gewogenen Uteri in mg/100g Körpergewicht.
Die Differenz der Mittelwerte der Uterusgewichte von Positiv- und Negativkontrolle wird als 100 % definiert. Eine Substanz mit antiöstrogener Wirkung verringert das gemessene Utersugewicht, wobei 100 % Wirkung eine Absenkung des Östradiol-stimulierten Uterusgewichtes auf den Wert der mit Vehikel behandelten Kontrolle bedeutet. Das Ergebnis wird als prozentuale Wirkung der jeweiligen Testsubstanz bei der getesteten Dosis/Tier/Tag ausgedrückt. Eine Substanz ist als Antiöstrogen um so wirksamer, je höher die prozentuale Aktivität bei möglichst niedriger Dosierung ist.

### 3. Untersuchungen zur knochenprotektiven Wirkung von östrogenen Partialagonisten

3 Monate alte weibliche Ratten werden ovarektomiert und unmittelbar nach der Operation für 28 Tage 1 mal täglich mit der Testverbindung behandelt. Die Applikation erfolgt subcutan in Arachisöl/Ethanol oder Benylbenzoat/ Rizinusöl. Die Tiere werden am Tag nach der letzten Applikation getötet und Tibia sowie Uterus entnommen. Die Uteri werden gewogen und für histologische Untersuchungen weiter aufgearbeitet. Die Bestimmung der Knochendichte erfolgt ex vivo an präparierten Tibien mittels pQCT (Quantitative Computertomographie) oder bildanalytisch an histologischen Längsschnitten. Die Bestimmung der Knochendichte erfolgt standardisiert in der sekundären Spongiosa in einem Abstand von 4-6 mm vom Gelenkkopf der proximalen Tibia durch jeweils drei Messungen.
Als positive Kontrolle wird entweder eine Gruppe von ovarektomierten Versuchstieren im gleichen Versuchsaufbau mit 0,3 µg 17-β Östradiol/Tier/Tag s.c. behandelt oder eine sham operierte intakte Gruppe von Tieren, die mit Vehikel behandelt wurde. Diese Tiere verlieren im Versuchszeitraum aufgrund ihrer Behandlung keine Knochenmasse. Als Negativkontrolle dient eine Gruppe von ovarektomierten Versuchstieren, die nur mit Vehikel behandelt werden und im Versuchszeitraum den maximal möglichen Knochenverlust erleiden. Der Unterschied zwischen der Knochendichte von Positiv- und Negativkontrolle wird als 100 % definiert. Eine Wirksubstanz wird in diesem Test durch die prozentuale knochenprotektive Wirkung bzw. die prozentuale Wirkung von 17-β Östradiol bei der jeweils getetsten Dosis beschrieben.

## Patentansprüche

1. Verwendung eines Knochenzellaktivators und eines Resorptionsinhibitors zur Herstellung eines Arzneimittels zur Behandlung oder Vorbeugung von postmenopausaler, seniler und sekundärer Osteoporose und zur Hormonersatztherapie, **dadurch gekennzeichnet, dass** der Knochenzellaktivator ein Vitamin-D-Metabolit oder ein Vitamin-D-Analogon und der Resorptionsinhibitor ein Östrogenpartialagonist ist und dass das Arzneimittel in einem Therapieplan verwendet wird, welcher einen oder mehrere Zyklen umfasst, wobei jeder Zyklus aus folgenden Schritten besteht:
a) tägliche Verabreichung eines Vitamin-D-Metaboliten oder Vitamin-D Analogons für 1-7 Tage.
b) anschließend daran oder nach einer Ruhephase bis zu 30 Tagen eine tägliche Verabreichung eines Östrogenpartialagonisten in einer Dosis in der der Östrogenpartialagonist im Uteruswachstumstest weniger als 50 % der Wirkung der Substitutionsdosis von 17-β-Östradiol und im Test auf Knochenwirkung mindestens 60 % der Wirkung einer Substitutionsdosis von 17-β-Ostradiol am Knochen hat für 21 -120 Tage.

2. Verwendung eines Knochenzellaktivators und eines Resorptionsinhibitors zur Herstellung eines Arzneimittels zur Behandlung oder Vorbeugung von postmenopausaler, seniler und sekundärer Osteoporose und zur Hormonersatztherapie, **dadurch gekennzeichnet, dass** der Knochenzellaktivator ein Vitamin-D-Metabolit oder ein Vitamin-D-Analogon und der Resorptionsinhibitor ein Östrogenpartialagonist ist und dass das Arzneimittel in einem Therapieplan verwendet wird, welcher einen oder mehrere Zyklen umfasst, wobei jeder Zyklus aus folgenden Schritten besteht:
a) tägliche Verabreichung eines Vitamin-D-Metaboliten und eines Östrogenpartialagonisten oder eines Vitamin-D-Analogons und eines Östrogenpartialagonisten für 1 - 7 Tage,
b) anschließend daran eine tägliche Verabreichung eines Östrogenpartialagonisten in einer Dosis in der der Östrogenpartialagonist im Uteruswachstumstest weniger als 50 % der Wirkung der Substitutionsdosis von 17-β-Östradiol und im Test auf Knochenwirkung mindestens 60 % der Wirkung einer Substitutionsdosis von 17-β-Ostradiol am Knochen hat für 21 - 120 Tage.

3. Verwendung nach Anspruch 1 oder 2, **dadurch gekennzeichnet, dass** der Östrogenpartialagonist aus der Gruppe bestehend aus Raloxifen, Tamoxifen, Hydroxytamoxifen, Clomiphencitrat, Droloxifen, Idoxifen, Centchroman, Levormeloxifen, Cyclophenyl, (S)-(+)-4-[7-(2,2-Dimethyl-1-oxopropoxy)-4-methyl-2-[4-[2-(1-piperidinyl)ethoxy]phenyl]-2H-1-benzopyran-3-yl]-phenyl-2,2-dimethylpropanoat (EM800), Lasofoxifen (CP336156), 14α,17α-Ethano-11β-[4-[5-(2-pyridinmethylsulfinyl)pentyloxy]phenyl]-1,3,5(10)-estratrien-3,17β-diol und 14α,17α-Ethano-11β-{-4-[5-(2-pyridinmethylsulfinyl)pentyloxy)phenyl}-1,3,5(10)-estratrien-3,17β-diol, (4,4,5,5,5-Pentafluorpentyl)-{5-[4-(6-phenyl-8,9-dihydro-7H-benzocyclohepten-5-yl)-phenoxy]-pentyl}-sulfid, S-{5-[6-Phenyl-8,9-dihydro-7H-benzocyclohepten-5-yl)-phenoxy]-pentyl}-thioacetat, 5-{4-[5-(4,4,5,5,5-Pentafluorpentansulfinyl)-pentyloxy]-phenyl}-6-phenyl-8,9-dihydro-7H-benzocyclohepten-2-ol, N-Butyl-N-methyl-2-{5-[4-(2-hydroxy-6-phenyl-8,9-dihydro-7H-benzocyclohepten-5yl)-phenoxy]-pentansulfinyl}-acetamid, 6-(4-Hydroxy-phenyl)5-{4-[5-(4,4,5,5,5-pentafluorpentansulfonyl)-pentyloxy]-phenyl}-8,9-dihydro-7H-benzocyclohepten-2-ol, 6-(4-Hydroxyphenyl)-5-{4-[5-(4,4,5,5,5-pentafluorpentan-sulfinyl)-pentyloxy]phenyl}-8,9-dihydro-7H-benzocyclohepten-2-ol, 5-{4-[4-(4,4,5,5,5-Pentafluorpentansulfinyl)-butyloxy]-phenyl}-6-phenyl-8,9-dihydro-7H-benzocyclohepten-2-ol, 6-Phenyl-5-{4-[6-(pyridin-2-ylmethansulfinyl)-hexyloxy]-phenyl}-8,9-dihydro-7H-benzocyclohepten-2-ol, 5-{4[5-(Furan-2-ylmethansulfinyl)-pentyloxy]phenyl}-6-phenyl-8,9-dihydro-7H-benzocyclohepten-2-ol, 5-{4-[5-(3,3,4,4,5,5,5-Heptafluorpentylthio)pentyloxy]-phenyl}-6-phenyl-8,9-dihydro-7H-benzocyclohepten-2-ol, 6-Phenyl-5-{4-[5-(4,4,4-triftuorbutylthio)-pentyloxy]-phenyl-8,9-dihydro-7H-benzocyclohepten-2-ol, 5-{4-5-Benzylsulfinyl-pentyloxy)-phenyl]6-phenyl-8,9-dihydro-7H-benzocyclohepten-2-ol, 6-Phenyl-5-{4-[5-(4-trifluormethylbenzylsulfinyl)pentyloxy]-phenyl}-8,9-dihydro-7H-benzocyclohepten-2-ol, 5-{4-[5-(4-tert.-Butylphenylthio)-pentyloxy]-phenyl)-6-phenyl-8,9-dihydro-7H-benzocylohepten-2-ol und 6-Phenyl-5-{4-[5-(4-trifluormethylphenylsulfinyl)-pentyloxy]-phenyl}-8,9-dihydro-7H-benzocyclohepten-2-ol ausgewählt ist.

4. Verwendung nach einem der Ansprüche 1 - 3, **dadurch gekennzeichnet, dass** der Vitamin-D-Metabolit oder das Vitamin-D-Analogon ausgewählt ist aus der Gruppe bestehend aus 25-Hydroxyvitamin D₃ oder D₂, 1α, 25-Dihydroxyvitamin D₃ oder D₂, 24R,25-Dihydroxyvitamin D₃, 1α,24R,25-Trihydroxyvitamin D₃, 1α-Hydroxyitamin D₃ oder D₂, 1α,24S-Dihydroxyvitamin D₂, 1α,24R-Dihydroxyvitamin D₃ oder D₂, 24-Epi-1α-Hydroxyvitamin D₂, 24-Epi-1α,25-Dihydroxy-Vitamin D₂, 19-Norvitamin D₂, 22-Oxa-1α,25-Dihydroxyvitamin D₃, 2β-(3-Hydroxypropyl)-1α,25-Dihydroxyvitamin D₃, 1α,25-Dihydroxy-16-en-23-in-vitamin D₃, 1α,25-Dihydroxy-26,26,26,27,27,27-hexafluor-16-en-23-vitamin D₃, (5Z,7E,22E,24E)(1S,3R)-24a,26,27-Trihomo-9,10-secopregna-5,7,10(19),22,24-pentaen-1,3,25-triol (EB 1089), (5Z,7E,22E)-(1S,3R,24R)-26,27-Cyclo-9,10-secocholesta-5,7,10(19),22-tetraen-1,3,24-triol (MC 903) und (5Z,7E)-(1S,3R,20R)-20-(2-Ethyl-2-hydroxyhexoxy)9,10-secopregna-5,7,10-(18)-trien-1,3-diol (KH 1060).

## Claims

1. Use of a bone cell activator and a resorption inhibitor for the production of a drug for the treatment or prophylaxis of postmenopausal, senile and secondary osteoporosis and for hormone replacement therapy, **characterized in that** the bone cell activator is a vitamin D metabolite or a vitamin D analogue and the resorption inhibitor is an oestrogen partial agonist and that the drug is used in a therapy plan which comprises one or several cycles, each cycle consisting of the following steps:
a) daily administration of a vitamin D metabolite or vitamin D analogue for 1-7 days,
b) following this or after a rest phase of up to 30 days, daily administration for 21-120 days of an oestrogen partial agonist at a dose at which the oestrogen agonist has less than 50% of the action of the substitution dose of 17-β-oestradiol in the uterus growth test and at least 60% of the action of a substitution dose of 17-β-oestradiol on bone in the test for bone action.

2. Use of a bone cell activator and a resorption inhibitor for the production of a drug for the treatment or prophylaxis of postmenopausal, senile and secondary osteoporosis and for hormone replacement therapy, **characterized in that** the bone cell activator is a vitamin D metabolite or a vitamin D analogue and the resorption inhibitor is an oestrogen partial agonist and that the drug is used in a therapy plan which comprises one or several cycles, each cycle consisting of the following steps:
a) daily administration of a vitamin D metabolite and an oestrogen partial agonist or a vitamin D analogue and an oestrogen partial agonist for 1-7 days,
b) following this, daily administration for 21-120 days of an oestrogen partial agonist at a dose at which the oestrogen agonist has less than 50% of the action of the substitution dose of 17-β-oestradiol in the uterus growth test and at least 60% of the action of a substitution dose of 17-β-oestradiol on bone in the test for bone action.

3. Use according to Claim 1 or 2, **characterized in that** the oestrogen partial agonist is selected from the group consisting of raloxifen, tamoxifen, hydroxytamoxifen, clomiphencitrate, droloxifen, idoxifen, centchroman, levormeloxifen, cyclophenyl, (S)-(+)-4-[7-(2,2-dimethyl-1-oxopropoxy)-4-methyl-2-[4-[2-(1-piperidinyl)ethoxy]phenyl]-2H-1-benzopyran-3-yl]-phenyl-2,2-dimethylpropanoate (EM800), lasofoxifene (CP336156), 14α,17α-ethano-11β-[4-[5-(2-pyridinmethylsulphinyl)pentyloxy]phenyl}-1,3,5(10)-oestratrien-3,17β-diol and 14α,17α-ethano-11β-{4-[5-(2-pyridinmethylsulphinyl)pentyloxy)phenyl}-1,3,5(10)-oestratrien-3,17β-diol, (4,4,5,5,5-pentafluorpentyl)-{5-[4-(6-phenyl-9,9-dihydro-7H-benzocyclohepten-5-yl)-phenoxy]-pentyl}-sulphide, S-{5-[6-phenyl-8,9-dihydro-7H-benzocyclohepten-5-yl)-phenoxy]-pentyl}-thioacetate, 5-{4-[5-(4,4,5,5,5-pentafluorpentansulphinyl)-pentyloxy]-phenyl}-6-phenyl-8,9-dihydro-7H-benzocyclohepten-2-ol, N-butyl-N-methyl-2-{5-[4-(2-hydroxy-6-phenyl-8,9-dihydro-7H-benzocyclohepten-5yl)-phenoxy]-pentansulphinyl}-acetamide, 6-(4-hydroxy-phenyl)5-{4-[5-(4,4,5,5,5-pentafluorpentansulphonyl)-pentyloxy]-phenyl}-8,9-dihydro-7H-benzocyclohepten-2-ol, 6-(4-hydroxyphenyl)-5-{4-(4,4,5,5,5-pentafluorpentan-sulphinyl)-pentyloxy]phenyl}-8,9-dihydro-7H-benzocyclohepten-2-ol, 5-{4-[4-(4,4,5,5,5-pentafluorpentansulphinyl)-butyloxy]-phenyl}-6-phenyl-8,9-dihydro-7H-benzocyclohepten-2-ol, 6-phenyl-5-{4-[6-(pyridin-2-ylmethansulphinyl)-hexyloxy]-phenyl}-8,9-dihydro-7H-benzocyclohepten-2-ol, 5-{4-[5-(furan-2-ylmethansulphinyl)-pentyloxy]phenyl}-6-phenyl-8,9-dihydro-7H-benzocyclohepten-2-ol, 5-{4-[5-(3,3,4,4,5,5,5-heptafluorpentylthio)pentyloxy]-phenyl}-6-phenyl-8,9-dihydro-7H-benzocyclohepten-2-ol, 6-phenyl-5-(4-[5-(4,4,4-trifluor-butylthio)-pentyloxy]-phenyl-8,9-dihydro-7H-benzocyclohepten-2-ol, 5-{4-5-benzylsulphinyl-pentyloxy)-phenyl]-6-phenyl-8,9-dihydro-7H-benzocyclohepten-2-ol, 6-phenyl-5-{4-[5-(4-trifluormethyl-benzylsulphinyl)- pentyloxy]-phenyl}-8,9-dihydro-7H-benzocyclohepten-2-ol, 5-{4-[5-(4-tert.-butylphenylthio)-pentyloxy]-phenyl}-6-phenyl-8,9-dihydro-7H-benzocyclohepten-2-ol and 6-phenyl-5-{4-[5-(4-trifluoromethyl-phenylsulphinyl)-pentyloxy]-phenyl}-8,9-dihydro-7H-benzocyclohepten-2-ol.

4. Use according to one of Claims 1-3, **characterized in that** the vitamin D metabolite or the vitamin D analogue is selected from the group consisting of 25-hydroxyvitamin D₃ or D₂, 1α,25-dihydroxyvitamin D₃ or D₂, 24R,25-dihydroxyvitamin D₃, 1α,24R,25-trihydroxyvitamin D₃, 1α-hydroxyvitamin D₃ or D₂, 1α,24S-dihydroxyvitamin D₂, 1α,24R-dihydroxyvitamin D₃ or D₂, 24-epi-1α-hydroxyvitamin D₂, 24-epi-1α,25-dihydroxyvitamin D₂, 19-nor-vitamin D₂, 22-oxa-1α,25-dihydroxyvitamin D₃, 2β-(3-hydroxypropyl)-1α,25-dihydroxyvitamin D₃, 1α,25-dihydroxy-16-en-23-yn-vitamin D₃, 1α,25-dihydroxy-26,26,26,27,27,27-hexafluor-16-en-23-vitamin D₃, (5Z,7E,22E,24E)-(1S, 3R)-24a,26,27-trihomo-9,10-secopregna-5,7,10 (19), 22,24-pentaen-1,3,25-triol (EB 1089), (5Z,7E,22E)-(1S,3R,24R)-26,27-cyclo-9,10-secocholesta-5,7,10(19),22-tetraen-1,3,24-triol (MC 903) and (5Z, 7E)-(1S,3R,20R)-20-(2-ethyl-2-hydroxyhexoxy)9,10-secopregna-5,7,10-(19)-trien-1,3-diol (KH 1060).

## Revendications

1. Utilisation d'un activateur de cellules osseuses et d'un inhibiteur de résorption, pour la fabrication d'un médicament destiné au traitement ou à la prévention de l'ostéoporose post-ménopausique, sénile et secondaire et pour l'hormonothérapie substitutive, **caractérisée en ce que** l'activateur de cellules osseuses est un métabolite de vitamine D ou un analogue de vitamine D et l'inhibiteur de résorption est un agoniste partiel d'oestrogène, et **en ce que** le médicament est utilisé dans un plan thérapeutique qui comprend un ou plusieurs cycles, chaque cycle consistant en les étapes suivantes :
a) administration journalière d'un métabolite de vitamine D ou d'un analogue de vitamine D, pendant 1-7 jours,
b) à la suite de cela ou après une phase de repos allant jusqu'à 30 jours, administration journalière pendant 21-120 jours d'un agoniste partiel d'oestrogène à une dose à laquelle l'agoniste partiel d'oestrogène a, dans le test de croissance utérine, moins de 50 % de l'effet de la dose substitutive de 17-β-estradiol et, dans le test d'action sur l'os, a sur l'os au moins 60 % de l'effet d'une dose substitutive de 17-β-estradiol.

2. Utilisation d'un activateur de cellules osseuses et d'un inhibiteur de résorption, pour la fabrication d'un médicament destiné au traitement ou à la prévention de l'ostéoporose post-ménopausique, sénile et secondaire et pour l'hormonothérapie substitutive, **caractérisée en ce que** l'activateur de cellules osseuses est un métabolite de vitamine D ou un analogue de vitamine D et l'inhibiteur de résorption est un agoniste partiel d'oestrogène, et **en ce que** le médicament est utilisé dans un plan thérapeutique qui comprend un ou plusieurs cycles, chaque cycle consistant en les étapes suivantes :
a) administration journalière pendant 1-7 jours d'un métabolite de vitamine D ou d'un analogue de vitamine D et d'un agoniste partiel d'oestrogène,
b) à la suite de cela, administration journalière pendant 21-120 jours d'un agoniste partiel d'oestrogène à une dose à laquelle l'agoniste partiel d'oestrogène a, dans le test de croissance utérine, moins de 50 % de l'effet de la dose substitutive de 17-β-estradiol et, dans le test d'action sur l'os, a sur l'os au moins 60 % de l'effet d'une dose substitutive de 17-β-estradiol.

3. Utilisation selon la revendication 1 ou 2, **caractérisée en ce que** l'agoniste partiel d'oestrogène est choisi dans le groupe constitué par le raloxifène, le tamoxifène, l'hydroxytamoxifène, le clomiphène citrate, le droloxifène, l'idoxifène, le centchromane, le lévorméloxifène, le cyclophényle, le 2,2-diméthylpropionate de (S)-(+)-4-[7-(2,2-diméthyl-1-oxopropoxy)-4-méthyl-2-[4-[2-(1-pipéridinyl)-éthoxy]phényl]-2H-1-benzopyrann-3-yl]phényle (EM 800) ; le lasofoxifène (CP 336156) ; le 14α,17α-éthano-11β-{4-[5-(2-pyridineméthylsulfinyl) pentyloxy]phényl}-1,3,5(10)-estratriène-3,17β-diol et le 14α,17α-éthano-11β-{4-[5-(2-pyridineméthylsulfinyl)pentyloxy]phényl}-1,3,5 (10)-estratriène-3,17β-diol, le (4,4,5,5,5-pentafluoropentyl)-{5-[4-(6-phényl-8,9-dihydro-7H-benzocycloheptén-5-yl)phénoxy]pentyl}sulfure, S-{5-[6-phényl-8,9-dihydro-7H-benzocycloheptén-5-yl)phénoxy]pentyl}thioacétate, le 5-{4-[5-(4,4,5,5,5-pentafluoropentanesulfinyl)pentyloxy]-phényl}-6-phényl-8,9-dihydro-7H-benzocycloheptén-2-ol, le N-butyl-N-méthyl-2-{5-[4-(2-hydroxy-6-phényl-8,9-dihydro-7H-benzocycloheptén-5-yl)-phénoxy]pentanesulfinyl}acétamide, le 6-(4-hydroxyphényl)-5-{4-[5-(4,4,5,5,5-pentafluoropentanesulfonyl)pentyloxy]phényl}-8,9-dihydro-7H-benzocycloheptén-2-ol, le 6-(4-hydroxyphényl)-5-{4-[5-(4,4,5,5,5-pentafluoropentanesulfinyl)pentyloxy]phényl}-8,9-dihydro-7H-benzocycloheptén-2-ol, le 5-{4-[4-(4,4,5,5,5-pentafluoropentanesulfinyl)-butyloxy]phényl}-6-phényl-8,9-dihydro-7H-benzocycloheptén-2-ol, le 6-phényl-5-{4-[6-(pyridin-2-yl-méthanesulfinyl)hexyloxy]phényl}-8,9-dihydro-7H-benzocycloheptén-2-ol, le 5-{4-[5-(furann-2-yl-méthanesulfinyl)pentyloxy]phényl}-6-phényl-8,9-dihydro-7H-benzocycloheptén-2-ol, le 5-{4-[5-(3,3,4,4,5,5,5-heptafluoropentylthio)-pentyloxy]phényl}-6-phényl-8,9-dihydro-7H-benzocycloheptén-2-ol, le 6-phényl-5-{4-[5-(4,4,4-trifluorobutylthio)pentyloxy]phényl}-8,9-dihydro-7H-benzocycloheptén-2-ol, le 5-{4-(5-benzylsulfinylpentyloxy)phényl}-6-phényl-8,9-dihydro-7H-benzocycloheptén-2-ol, le 6-phényl-5-{4-[5-(4-trifluorométhylbenzylsulfinyl)pentyloxy]phényl}-8,9-dihydro-7H-benzocycloheptén-2-ol, le 5-{4-[5-(4-tert-butylphénylthio)pentyloxy]phényl]-6-phényl-8,9-dihydro-7H-benzocycloheptén-2-ol et le 6-phényl-5-{4-[5-(4-trifluorométhylphénylsulfinyl)pentyloxy]phényl}-8,9-dihydro-7H-benzocycloheptén-2-ol.

4. Utilisation selon l'une quelconque des revendications 1 à 3, **caractérisée en ce que** le métabolite de vitamine D ou l'analogue de vitamine D est choisi dans le groupe constitué par la 25-hydroxyvitamine D₃ ou D₂, la 1α,25-dihydroxyvitamine D₃ ou D₂, la 24R,25-dihydroxyvitamine D₃, la 1α,24R,25-trihydroxyvitamine D₃, la 1α-hydroxyvitamine D₃ ou D₂, la 1α,24S-dihydroxyvitamine D₂, la 1α,24R-dihydroxyvitamine D₃ ou D₂, la 24-épi-1α-hydroxyvitamine D₂, la 24-épi-1α,25-dihydroxyvitamine D₂, 19-norvitamine D₂, la 22-oxa-1α,25-dihydroxyvitamine D₃, la 2β-(3-hydroxypropyl)-1α-25-dihydroxyvitamine D₃, la 1α,25-dihydroxy-16-én-23-ine-vitamine D₃, la 1α,25-dihydroxy-26,26,26,27,27,27-hexafluor-16-ène-23-vitamine D₃, le (5Z,7E,22E,24E)-(18,3R)-24a,26,27-trihomo-9,10-sécoprégna-5,7,10 (19), 22, 24-pentaène-1,3,25-triol (EB 1089), le (5Z, 7E, 22E)-(1S, 3R, 24R)-26,27-cyclo-9,10-sécocholesta-5,7,10(19),22-tétraène-1,3,24-triol (MC 903) et le (5Z,7E)-(1S,3R,20R)-20-(2-éthyl-2-hydroxyhexoxy)-9,10-sécoprégna-5,7,10(19)-triène-1,3-diol (KH 1060).
